# EUROPEAN PATENT SPECIFICATION

(11) **EP 0 901 460 B1**
(45) Date of publication and mention of the grant of the patent: **18.09.2002**
(21) Application number: 97952841.1
(22) Date of filing: 01.12.1997
(51) Int. Cl.: C07C 231/02, C07C 237/46, A61K 49/04

(54) **A PROCESS FOR THE PREPARATION OF 5-AMINO-2,4,6-TRIIODO-1,3-BENZENEDICARBOXYLIC ACID DERIVATIVES**
VERFAHREN ZUR HERSTELLUNG VON 5-AMINO-2,4,6-TRIIOD-BENZOLDICARBONSÄUREDERIVATE
PROCEDE DE PREPARATION DE DERIVES DE L'ACIDE 5-AMINO-2,4,6-TRIIODO-1,3-BENZENEDICARBOXYLIQUE

(30) Priority: 04.12.1996 IT MI962545
(43) Date of publication of application: 17.03.1999
(73) Proprietor: BRACCO IMAGING S.p.A., 20134 Milano (IT)
(72) Inventor: DESANTIS, Nicola, I-20020 Ceriano Laghetto (IT)
(74) Representative: Minoja, Fabrizio, Dr.
(86) International application number: EP9706720
(87) International publication number: WO98024757

(56) References cited:
- WO-A-93/16981
- GB-A- 1 472 050
- US-A- 5 075 502

## Description

This invention refers to a process for the preparation of (S)-N,N'-bis[2-hydroxy-1-(hydroxymethyl)ethyl]-5-[(2-hydroxy-1-oxopropyl)amino]-2,4,6-triiodo-1,3-benzenedicarboxamide (III)., in a single step, starting from 5-amino-2,4,6-triiodo-1,3-benzenedicarboxylic acid dichloride (compound of formula I), according to the reaction pathway of Scheme 1.

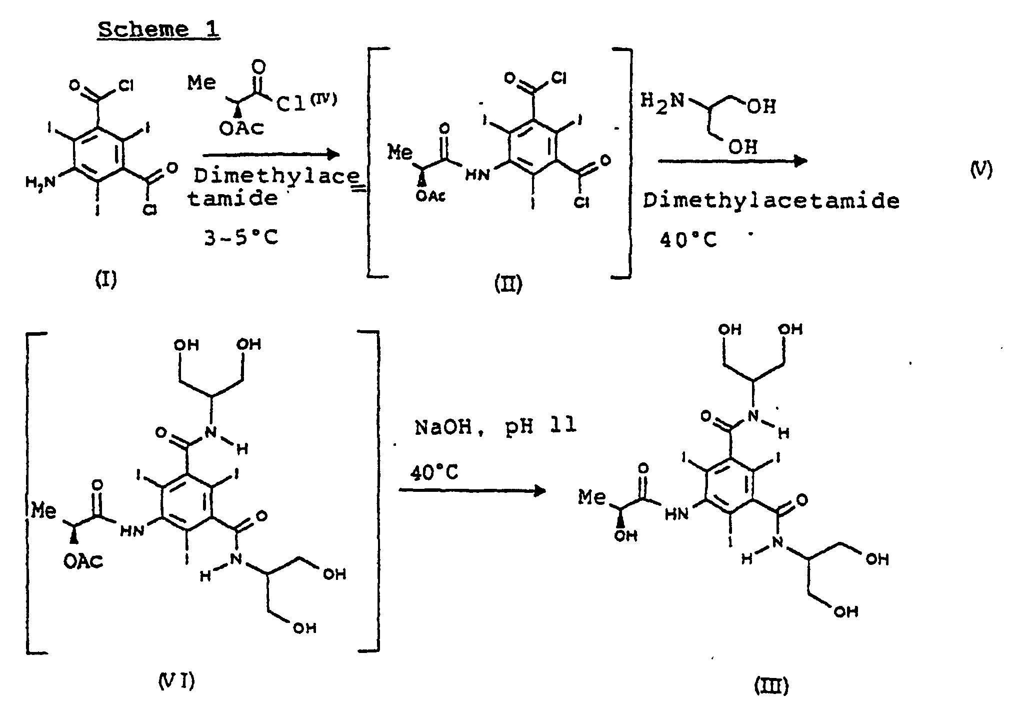

The compound of formula (III), better known as Iopamidol, is one of the best-selling X-ray non-ionic contrast media all over the world and its synthesis was described in patent GB 1472050.

Said synthesis foresees the reaction steps already shown in Scheme 1, but it involves the isolation of intermediate (II), whose purification is needed for the following reasons:
- the presence of hydrochloric acid as the reaction byproduct which can react in the successive step with 2-amino-1,3-propanediol (serinol);
- the presence of the excess of (S)-(-)-[2-(acetyloxy)]propionic acid chloride which can also react with serinol;
- the need for removing the acid by-products deriving from 5-amino-2,4,6,-triiodobenzoic acid.

We have surprisingly found, and it is the object of this invention, that, in the process of scheme 1 above, the addition of the tertiary amine of general formula NR₁R₂R₃, wherein:
- R₁, R₂, R₃: are independently a (C₁-C₁₂) straight or branched alkyl group, or (C₁-C₃)alkyl(C₆-C₁₀) aryl or a (C₁-C₄)alkenyl group,
in the formation step of compound (II) starting from compound (I), allows to hydrolize and neutralize the excess of (s)-(-)-[2-(acetyloxy)]propionic acid chloride (IV) so that to make it inert, therefore the synthetic process can be continued, without isolating compound (II).

The amine is added in an amount equal to 3-15% excess on the basis of the molar amount of (S)-(-)-[2-(acetyloxy)]propionic acid chloride (IV) of the reaction.

Particularly preferred are the amines substituted by straight alkyl groups, particularly triethylamine, tripropylamine and tributylamine, diisopropylethylamine, or, among the mixed-structure amines, preferred are those substituted by alkylaromatic chains, such as a benzyl group, for instance diethylbenzylamine, or with alkyl chains containing double bonds, for instance the allyl group.

The process of this invention is particularly advantageous since the synthetic process of the compound of formula (III) is made quite easier from an industrial point of view.

The amine addition is carried out by diluting the solution containing reactants (II) and (IV) to the concentration necessary for the successive step.

The addition is carried out slowly so that the reaction mixture temperature is comprised between 0°C and 30°C, to avoid that hydrolysis and neutralization heat of the acid compounds in the reaction cause the formation of undesired by-products. Generally, the addition time is comprised between 2 and 8h.

The hydrolysis of the excess of the compound of formula (IV) is carried out either using the necessary amount of water which is contained in the added tertiary amine, or water is directly added to the solution in case the amine is anhydrous.

As previously described, the amine is added in a 3-15% excess on the basis of the reaction stoichiometry, to secure the quantity of water necessary to the hydrolysis.

The use of inorganic bases is not equally suitable since they will react very slowly due to their insolubility in the organic solutions. In addition, the salts resulting from the hydrolysis of the compound (IV) are not easily filterable and washable, thus causing yield losses.

On the contrary, the organic bases which we use are mixable with the final solution of the compound of formula (II), producing a hydrolysis and neutralization of the compound of formula (IV) in a very short time.

A particularly preferred base is triethylamine, as it forms a hydrochloride which is approximately insoluble by 90% in the solution. The precipitated hydrochloride is filtered and easily washed with the reaction solvent (N,N-dimethylacetamide, DMAC).

Therefore the use of tertiary amines allows to perform the next step without isolating compound (II), avoiding any formation of by-products due to the reactivity of compound (IV) with the amino and hydroxyl groups of the aromatic nucleus.

In addition, the same tertiary amines, already present in the reaction, can act as bases in the successive step between compound (II) and 2-amino-1,3-propanediol (V) to give compound (VI), as already described in patent GB 1472050, without need for an excess of compound (V).

DMAC in the reaction medium is distilled off under vacuum before carrying out the hydrolysis reaction to give compound (III), and the resulting residue is diluted with water.

Various methods can be used to recovery the tertiary amines used in the synthetic steps of Scheme 1, and they comprise the following purification techniques:
1) Formation of compound (VI) starting from compound (II).
   The salts formed between the tertiary amine and (S)-(-)-[2-(acetyloxy)]propionic acid, acetic acid, lactic acid and hydrochloric acid are eluted on a series of ion exchangers constituted by a column containing a H+ or Na+ form cation exchanger of the Amberjet® 1200 type, and an anion exchanger of the Relite® MG 1 type; so that the resulting solution, obtained after amidation, is desalted removing both the salts generated by the amidation reaction and those produced by the synthetic reaction of the compound of formula (II).
   Alternatively, a mixed bed of ion exchangers having the same features can be used. Anion or cation exchangers with similar characteristics from different manufacturers can be used, for example gel or polystyrene-based macroporous exchangers with sulphonic acid groups.
2) Hydrolysis of compound (VI) to give compound (III)
   In this case the hydrolysis, as previously described, is effected to deprotect the hydroxyl group at the 5-position of the aromatic ring.
   The amines can be recovered through a simple separation of the layers where the organic one is that containing the tertiary amine or through water azeotropic distillation, when the used amines allow it.

The process of the invention avoids a synthetic step, thus making the preparation of the compound of formula (III) in higher yields and in an useful way on industrial scale possible.

The process of this invention is generally applicable to non-ionic iodinated contrast media deriving from 5-amino-2,4,6-triiodo-1,3-benzenedicarboxylic acid having an acylamino residue at the 5- position. Therefore, it is a further object of this invention a process for the preparation of said non-ionic contrast media which, without isolating any intermediate, comprises the following steps:
a) reacting 5-amino-2-4-6-triiodo-1,3-benzenedicarboxylic acid dichloride with an acyl chloride, containing hydroxy groups protected as ester, to give a solution of 5-acylamino-2,4,6-triiodo-1,3-benzenedicarboxylic acid dichloride in dimethylacetamide;
b) adding the crude solution from step a) with a tertiary amine of formula NR₁R₂R₃, wherein R₁, R₂, R₃ are independently a (C₁-C₁₂) straight or branched alkyl group, (C₁-C₃) alkyl(C₆-C₁₀)aryl group, (C₁-C₄)alkenyl group, in a 3-15% excess amount on the basis of the molar amount of acyl chloride, keeping the reaction temperature between 0 and 30°C;
c) adding the solution from step b) with an aminoalcohol to give the corresponding N,N'-bis[2,4,6-triiodo-1,3-benzenedicarboxamide], which is not isolated but directly hydrolyzed thereby deprotecting the protected hydroxy groups.

Examples of said contrast media are Iopromide, Iosimide, Iotriside, Ioversol, Iohexol, Iopentol, Ioxilan, Iobitridol, Iomeprol.

In particular, Iopromide (whose preparation is described in DE 2909439), can be prepared with the process of the invention by adding the amine of the invention in the step of reaction between 5-amino-2,4,6-triiodoisophtalic acid dichloride and methoxyacetic acid and then the formation of the amides with, first, 2,3-dihydroxy-propylamine and, second, 2,3-dihydroxy-N-methylpropylamine without isolation of the intermediates.

Similarly, Iobitridol (whose preparation is disclosed in US 5043152) can be prepared with the process of the invention by adding the amine of the invention in the step of reaction between 5-amino-2,4,6-triiodoisophthalic acid dichloride and 2-isopropyl-1,3-dioxane-5-carbonyl chloride and then the formation of the diamide with N-methyl-2-aminoethanol.

Iohexol can be prepared with the process of the invention following the reaction scheme described in Acta Pharm. Sue. 20, 219, 1983, by adding the amine of the invention in the step of reaction between 5-amino-2,4,6-triiodoisophthalic acid dichloride and acetyl chloride and then the formation of the diamide with 3-methylamino 1,2-propanediol.

Iomeprol can be prepared with the process of the invention following the reaction scheme described in EP 26281, by adding the amine of the invention in the step of reaction between 5-amino-2,4,6-triiodoisophtalic acid dichloride and acetoxyacetyl chloride and then the formation of the diamide with 1,3-dihydroxyisopropylamine.

The following examples are intended to illustrate the best experimental conditions to carry out the process of this invention.

### EXAMPLE 1

### Preparation of a crude solution of (S)-5-[[2-(acetyloxy)-1-oxopropyl]amino]-2,4,6-triiodo-1,3-benzenedicarboxylic acid dichloride (II) in DMAC

700 g of 5-amino-2,4,6-triiodo-1,3-benzenedicarboxylic acid dichloride (prepared according to the procedure described in GB 1472050) are dissolved in 1 kg of DMAC at room temperature and under stirring. 288 g of (S)-(-)-[2-(acetyloxy)]propionic acid chloride (prepared according to GB 1472050) are then added in 4h, keeping temperature between 8-15°C. The reaction is complete after keeping it for 30-40 h at a temperature between 6-15°C.

### EXAMPLE 2

### (S)-N,N'-bis[(2-hydroxy-1-hydroxymethyl)ethyl]-5-[[(2-hydroxy-1-oxopropyl] amino]-2,4,6-triodo-1,3-benzenedicarboxamide (III)

680 g of the solution of Example 1 containing respectively: 285.6 g of compound (II), 19.8 g of hydrochloric acid, 37.6 g of (S)-(-)-[2-(acetyloxy)] propionic acid chloride, and 337 g of DMAC are cooled to a temperature of 0-5°C.

105.6 g of 100% triethylamine are slowly added to neutralize hydrochloric acid and any acid compounds deriving from hydrolysis of (S)-(-)-[2-(acetyloxy)] propionic acid chloride.

When the addition is completed, the mixture is kept under stirring at a temperature of 0-5°C to promote the precipitation of triethylamine hydrochloride. The precipitated triethylamine hydrochloride is filtered and washed with 600 g of DMAC. The solution is cooled to a temperature below 5°C and 153 g of 2-amino-1,3-propanediol are added.

The reaction is completed after 8h keeping the solution at a temperature between 10-15°C. Most of the reaction solvent is distilled at 95°C and 10 mbar to give a viscous residue, which is diluted with deionized water while hot.

The resulting solution is eluted on 800 mL of the H⁺-form cation exchanger Amberjet® 1200 and 650 mL of the free base-form Relite® MG 1 forming a series of layered beds with 1.5 L of deionized water.

The resulting solution is brought to 35°C and 120 g of 30% (w/w) sodium hydroxide are added, stirring for 7h to hydrolyze the acetic ester of the desired product. Then pH is adjusted to 6.5 with 50 g of 34% (w/w) hydrochloric acid to stop the hydrolysis.

The resulting crude solution is then treated according to the procedure described in GB 1472050.

Yield in compound of formula (III) starting from compound of formula (I) = 78%.

¹H-NMR, ¹³C-NMR, IR e MS spectra are consistent with literature values.

### EXAMPLE 3

### Alternative to EXAMPLE 2.

680 g of the solution prepared in Example 1 containing respectively: 285.6 g of compound (II), 19.8 g of hydrochloric acid, 37.6 g of (S)-(-)-[2-(acetyloxy)] propionic acid chloride, and 337 g of DMAC are cooled to a temperature of 0-5°C.

190.5 g of 100% triethylamine diluted in 400 g of DMAC are slowly added to neutralize hydrochloric acid and any acid compounds deriving from the hydrolysis of (S)-(-)-[2-(acetyloxy)]propionic acid chloride. When the addition is completed, the mixture is kept under stirring at a temperature of 0-5°C to promote the precipitation of triethylamine hydrochloride. The precipitated triethylamine hydrochloride is filtered and washed with 600 g of DMAC.

The solution is cooled to a temperature below 5°C and 84 g of 2-amino-1,3-propanediol are added. The reaction is completed after 8h keeping the solution temperature between 10-15°C.

The precipitated triethylammonium chloride is filtered and washed with 200 g of DMAC.

Most of the reaction solvent is distilled at 95°C and 10 mbar to give a viscous residue, which is diluted with deionized water while hot.

The resulting solution is eluted on 800 mL of the H⁺-form cation exchanger Amberjet® 1200 and 650 mL of the free base-form Relite® MG 1 forming a series of layered mixed beds with 1.5 L of deionized water.

The resulting solution is brought to 35°C and 120 g of 30% (w/w) sodium hydroxide are added, stirring for 7h to hydrolyze the acetic ester of the desired product. Then pH is adjusted to 6.5 with 50 g of 34% (w/w) hydrochloric acid to stop the hydrolysis.

The resulting crude solution is treated according to the method described in GB 1472050.

Yield in compound of formula (III) starting from compound of formula (I)= 85%.

### EXAMPLE 4

### Alternative to EXAMPLE 2

680 g of the solution prepared in Example 1 containing respectively: 285.6 g of compound (II), 19.8 g of hydrochloric acid, 37.6 g of (S)-(-)-[2-(acetyloxy)] propionic acid chloride, and 337 g of DMAC are cooled to a temperature of 0-5°C.

269.7 g of 100% tripropylamine diluted in 400 g of DMAC are slowly added to neutralize hydrochloric acid and any acid compounds deriving from the hydrolysis of (S)-(-)-[2-(acetyloxy)]propionic acid chloride.

The solution is cooled to a temperature below 5°C and 84 g of 2-amino-1,3-propanediol are added. The reaction is completed after 8h keeping the solution at a temperature between 10-15°C.

Most of the reaction solvent is distilled at 95°C and 10 mbar to give a viscous residue, which is diluted with deionized water while hot.

The resulting solution is brought to 35°C and 371 g of 30% (w/w) sodium hydroxide are added, stirring for 7h to hydrolyze the acetic ester of the desired product. When the reaction is completed, the upper organic layer containing tripropylamine and traces of DMAC is discarded. The aqueous phase is the crude solution of the compound of formula (III) which is treated according to the procedure described in GB 1472050.

Yield in compound of formula (III) starting from compound of formula (I)= 81%.

### EXAMPLE 5

### Alternative to EXAMPLE 4

680 g of solution prepared in Example 1 containing respectively: 285.6 g of (S)-5-[[2-(acetyloxy)-1-oxopropyl ]amino]-2,4,6-triiodo-1,3-benzenedicarboxylic acid, 19.8 g of hydrochloric acid, 37.6 g (S)-(-)-[2-(acetyloxy)] propionic acid chloride, and 337 g of DMAC are cooled to a temperature of 0-5°C.

269.7 g of 100% tripropylamine diluted in 400 g of DMAC are slowly added to neutralize hydrochloric acid and any acid compounds from the hydrolysis of (S)-(-)-[2-(acetyloxy)]propionic acid chloride.

The solution is cooled to a temperature below 5°C and 84 g of 2-amino-1,3-propanediol are added. The reaction is completed after 8h, keeping the solution temperature between 10-15°C. Most of the reaction solvent is distilled at 95°C and 10 mbar to give a viscous residue, which is diluted with deionized water while hot.

The resulting solution is eluted on 1000 mL of the H⁺-form cation exchanger Amberjet® 1200 and 850 mL of free base-form anion exchanger Relite® MG 1 composing a mixed bed of cation and anion exchangers with 1.5 L of deionized water.

The resulting solution is warmed to 35°C and 120 g of 30% (w/w) sodium hydroxide are added, stirring for 7h to hydrolyze the acetic ester of the desired product. pH is adjusted to 6.5 with 50 g of 34% (w/w) hydrochloric acid to stop hydrolysis.

The resulting crude solution is treated according to the method described in GB 1472050.

Yield in compound of formula (III) starting from compound of formula (I)= 79%.

## Claims

1. A process for the preparation of non-ionic iodinated contrast media derivatives of 5-amino-2,4,6-Triiodo-1,3-benzenedicarboxylic acid and having a hydroxy-substituted acylamino residue at the 5- position, comprising the following steps without isolating any intermediate:
a) reacting 5-amino-2,4,6-triiodo-1,3-benzenedicarboxylic acid dichloride with an acyl chloride, containing hydroxy groups protected as ester, to give a solution of 5-acylamino-2,4,6-triiodo-1,3-benzenedicarboxylic acid dichloride in dimethylacetamide;
b) adding the crude solution resulting from step a) with a tertiary amine of formula NR₁R₂R₃, wherein R₁, R₂, R₃ are independently a (C₁-C₁₂) straight or branched alkyl group, (C₁-C₃)alkyl(C₆-C₁₀)aryl group, (C₁-C₄)alkenyl group, in a 3-15% excess amount on the basis of the molar amount of acyl chloride, keeping the reaction temperature between 0 and 30°C;
c) adding to the solution resulting from step b) an aminoalcohol to give the corresponding N,N'-bis[2,4,6-triiodo-1,3-benzenedicarboxamide] which is directly hydrolyzed to deprotect the protected hydroxy groups.

2. A process according to claim 1, in which said contrast medium is selected from the group consisting of: Iopromide, Iosimide, Ioversol, Iotriside, Iohexol, Iopentol, Ioxilan, Iobitridol, Iomeprol.

3. A process according to claim 1 for the preparation of (S)-N,N'-bis[2-hydroxy-1-(hydroxymethyl)ethyl]-5-[(2-hydroxy-1-oxopropyl)amino]-2,4,6-triiodo-1,3-benzenedicarboxamide, without isolation of any intermediate, comprising the following steps of Scheme 1: 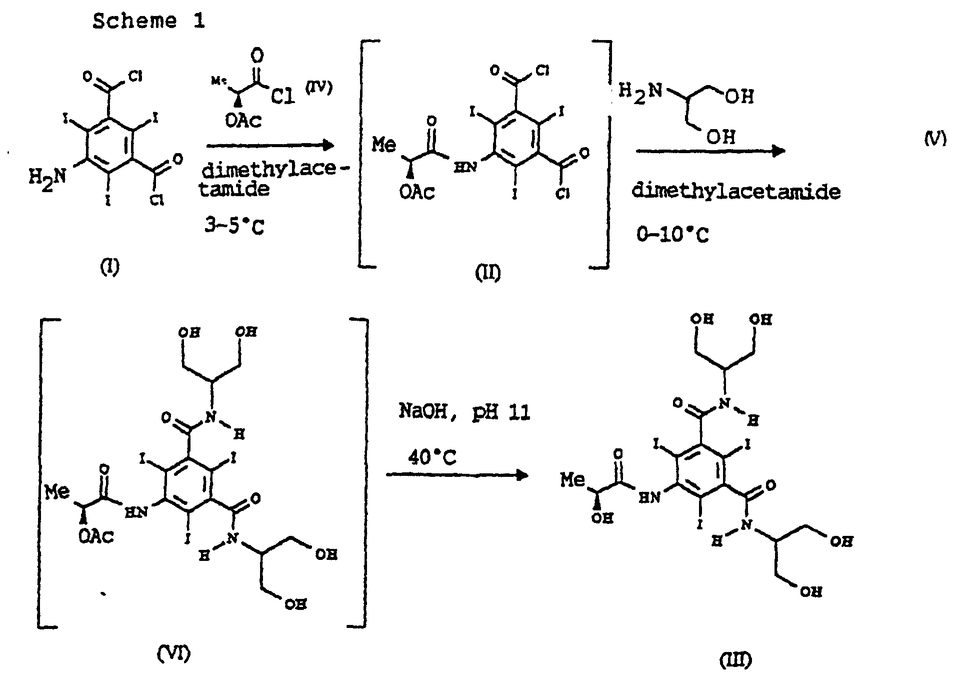
a) reacting compound (I), 5-amino-2,4,6-triiodo-1,3-benzenedicarboxylic acid dichloride, with S-(-)-[2-(acetyloxy)]propionic acid chloride in dimethylacetamide at a temperature of 3-5°C, to give a solution of the compound of formula (II), (S)-5-[[2-(acetyloxy)-1-oxopropyl]amino]-2,4,6-triiodo-1,3-benzenedicarboxylic acid dichloride in dimethylacetamide;
b) adding a tertiary amine of general formula NR₁R₂R₃ to the crude solution resulting from step a), wherein
R₁, R₂, R₃ are independently a (C₁-C₁₂) straight or branched alkyl group, (C₁-C₃)alkyl(C₆-C₁₀)aryl group, (C₁-C₄)alkenyl group,
in a 3-15% excess amount on the basis of the molar amount of (S)-(-)-[2-(acetyloxy)]propionic acid chloride (IV) of the reaction and in a time interval such that to keep the temperature of the reaction mixture between 0 and 30°C;
c) adding solid 2-amino-1,3-propanediol to the solution resulting from step b), at a temperature of 0-10, during 5 to 20h, to give a solution of the compound (VI), which, without being isolated, is transformed into compound (III) after removal of the solvent, dilution with water and optional chromatographic purification on cation and anion exchangers and hydrolysis reaction under basic conditions by addition of 30% NaOH, at pH 11, for 5-10 h;
d) isolating compound of formula (III).

4. A process according to claim 1 or 3, in which the tertiary amines of general formula NR₁R₂R₃ are selected from the group consisting of: triethylamine, tripropylamine, tributylamine, diethylbenzylamine, diisopropylethylamine.

5. A process according to claim 4, in which the tertiary amine of general formula NR₁R₂R₃ is triethylamine.

6. A process according to claim 5, in which triethylamine, when the amidation reaction is completed, is filtered off as triethylamine hydrochloride.

7. A process according to claim 1 or 3, in which the amine addition is carried out by diluting the solution to the desired concentration for the successive step at a temperature comprised between 0°C and 30°C, during 2 - 8h.

8. A process according to claim 1 or 3, in which the added tertiary amine is recovered at the end of the formation step of the compound (VI) starting from compound (II) through elution of the crude solution on a series of ion exchangers constituted by a column containing the cation exchangers Amberjet® 1200 or its equivalent and a column containing the anion exchangers Relite® MG 1 or its equivalent.

9. A process according to claim 8, in which the series of ion exchangers is substituted by a mixed bed of ion exchangers with the same characteristics.

10. A process according to claim 1 or 3, in which the added tertiary amine is recovered during the hydrolysis step of product (VI) to give compound (III), performing a separation of the layers where the organic one corresponds to that containing the tertiary amine.

11. A process according to claim 10, in which the added tertiary amines are recovered through azeotropic water distillation, when the amines allow it.

## Patentansprüche

1. Verfahren zur Herstellung von nicht-ionischen iodierten Kontrastmedien-Derivaten von 5-Amino-2,4,6-triiodo-1,3-benzoldicarbonsäure mit einem Hydroxysubstituierten Acylaminorest in 5-Position, umfassend die folgenden Schritte ohne Isolierung irgendwelcher Zwischenprodukte:
a) Umsetzung von 5-Amino-2,4,6-triiodo-1,3-benzoldicarbonsäuredichlorid mit einem Acylchlorid, das als Ester geschützte Hydroxygruppen enthält, so daß eine Lösung von 5-Acylamino-2,4,6-triiodo-1,3-benzoldicarbonsäuredichlorid in Dimethylacetamid erhalten wird;
b) Zugabe eines tertiären Amins der Formel NR₁R₂R₃, wobei R₁, R₂ und R₃ unabhängig voneinander eine geradkettige oder verzweigte (C₁-C₁₂)Alkylgruppe, (C₁-C₃)Alkyl(C₆-C₁₀)arylgruppe, (C₁-C₄)Alkenylgruppe ist, in einer Überschußmenge von 3 bis 15 %, bezogen auf die molare Menge an Acylchlorid, zu der aus Stufe a) resultierenden rohen Lösung, wobei die Reaktionstemperatur zwischen 0 und 30°C gehalten wird;
c) Zugabe eines Aminoalkohols zu der aus Schritt b) resultierenden Lösung, so daß das entsprechende N,N'-Bis[2,4,6-triiodo-1,3-benzoldicarboxamid] erhalten wird, das direkt zur Entschützung der geschützten Hydroxygruppen hydrolysiert wird.

2. Verfahren nach Anspruch 1, bei dem das Kontrastmedium ausgewählt wird aus der Gruppe, die besteht aus: lopromid, losimid, loversol, lotrisid, lohexol, lopentol, loxilan, lobitridol, lomeprol.

3. Verfahren nach Anspruch 1 zur Herstellung von (S)-N,N'-Bis[2-hydroxy-1-(hydroxymethyl)ethyl]-5-[(2-hydroxy-1-oxopropyl)amino]-2,4,6-triiodo-1,3-benzoldicarboxamid ohne Isolierung irgendwelcher Zwischenprodukte, umfassend die folgenden Stufen von Schema 1: 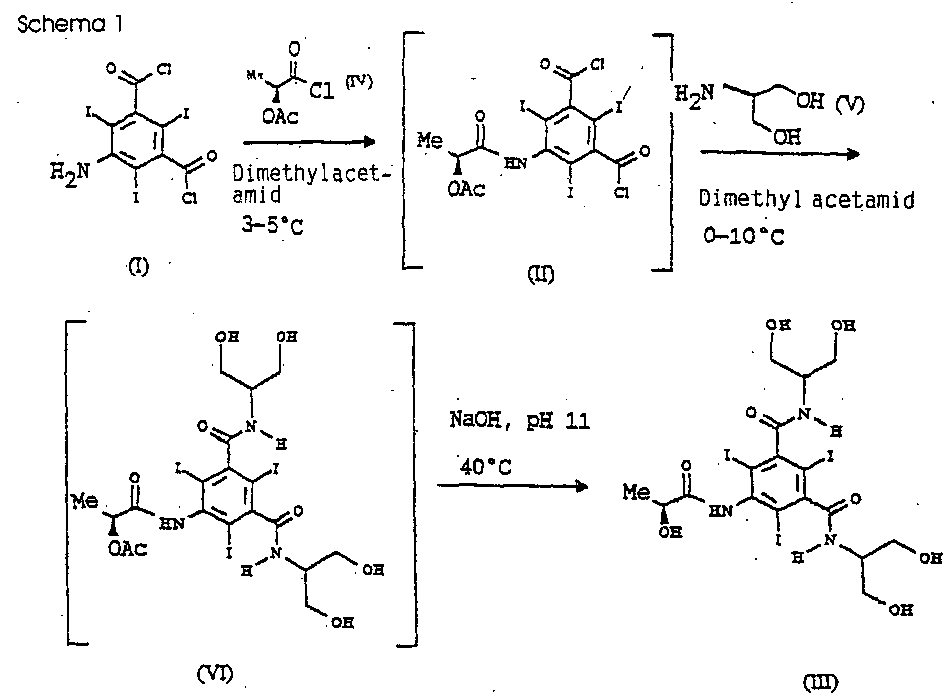
a) Umsetzung von Verbindung (I), 5-Amino-2,4,6-triiodo-1,3-benzoldicarbonsäuredichlorid, mit S-(-)-[2-(Acetyloxy)]propionsäurechlorid in Dimethylacetamid bei einer Temperatur von 3 - 5°C zur Herstellung einer Lösung der Verbindung der Formel (II), (S)-5-[[2-(Acetyloxy)-1-oxopropyl]amino]-2,4,6-triiodo-1,3-benzoldicarbonsäuredichlorid in Dimethylacetamid;
b) Zugabe eines tertiären Amins der allgemeinen Formel NR₁R₂R₃ zu der aus Schritt a) resultierenden rohen Lösung, wobei R₁, R₂, R₃ unabhängig voneinander eine geradkettige oder verzweigte (C₁-C₁₂)-Alkylgruppe, (C₁-C₃)Alkyl(C₆-C₁₀)arylgruppe, (C₁-C₄)Alkenylgruppe ist, in einem 3 bis 15 %igen Überschuss bezogen auf die molare Menge von (S)-(-)-[2-Acetyloxy)]propionsäuredichlorid (IV) in der Reaktion und in einem solchen Zeitintervall, daß die Temperatur der Reaktionsmischung zwischen 0 und 30°C gehalten wird;
c) Zugabe von festem 2-Amino-1,3-propandiol zu der aus Schritt b) resultierenden Lösung bei einer Temperatur von 0 bis 10°C während 5 bis 20 Stunden zur Herstellung einer Lösung der Verbindung (VI), die, ohne isoliert zu werden, nach Entfernung des Lösungsmittels, Verdünnen mit Wasser und gegebenenfalls chromatographischer Reinigung auf Kationen- und Anionenaustauschern und Hydrolysereaktion unter basischen Bedingungen durch Zugabe von 30 % NaOH bei pH 11 während 5 bis 10 Stunden in Verbindung (III) umgewandelt wird;
d) Isolierung der Verbindung der Formel (III).

4. Verfahren nach Anspruch 1 oder 3, bei dem die tertiären Amine der allgemeinen Formel NR₁R₂R₃ ausgewählt werden aus der Gruppe, die besteht aus: Triethylamin, Tripropylamin, Tributylamin, Diethylbenzylamin, Dlisopropylethylamin.

5. Verfahren nach Anspruch 4, bei dem das tertiäre Amin der allgemeinen Formel NR₁R₂R₃ Triethylamin ist.

6. Verfahren nach Anspruch 5, bei dem Triethylamin nach Beendigung der Amidierungsreaktion als Triethylaminhydrochlorid abfiltriert wird.

7. Verfahren nach Anspruch 1 oder 3, bei dem die Aminzugabe durch Verdünnen der Lösung auf die gewünschte Konzentration wie im nachfolgenden Schritt bei einer Temperatur zwischen 0°C und 30°C während 2 bis 8 Stunden durchgeführt wird.

8. Verfahren nach Anspruch 1 oder 3, bei dem das zugegebene tertiäre Amin am Ende des Bildungsschritts der Verbindung (VI) ausgehend von Verbindung (II) durch Elution der rohen Lösung auf einer Reihe von Ionenaustauschern wiedergewonnen wird, die gebildet wird von einer Säule, die die Kationenaustauscher Amberjet® 1200 oder deren Äquivalent und einer Säule, die die Anionenaustauscher Relite® MG 1 oder deren Äquivalent enthält.

9. Verfahren nach Anspruch 8, bei dem die Reihe von Ionenaustauschem durch ein Mischbett von Ionenaustauschern mit denselben Elgenschaften ersetzt wird.

10. Verfahren nach Anspruch 1 oder 3, bei dem das zugegebene tertiäre Amin während des Hydrolyseschritts von Produkt (VI) zur Herstellung von Verbindung (III) durch Durchführung einer Trennung der Schichten isoliert wird, wobei die organische Schicht derjenigen entspricht, die das tertiäre Amin enthält.

11. Verfahren nach Anspruch 10, bei dem die zugegebenen tertiären Amine durch azeotrope Wasserdestillation wiedergewonnen werden, wenn die Amine dies ermöglichen.

## Revendications

1. Procédé pour la préparation de milieux de contraste constitués de dérivés iodés non ioniques d'acide 5-amino-2,4,6-triiodo-1,3-benzènedicarboxylique et ayant un résidu acylamino hydroxylé en position 5, comprenant les étapes suivantes, sans isoler aucun produit intermédiaire, consistant :
a) à faire réagir du dichlorure d'acide 5-amino-2,4,6-triiodo-1,3-benzènedicarboxylique avec un chlorure d'acyle contenant des groupes hydroxy protégés sous forme ester, pour donner une solution de dichlorure d'acide 5-acylamino-2,4,6-triiodo-1,3-benzènedicarboxylique dans du diméthylacétamide ;
b) à ajouter à la solution brute résultant de l'étape a) une amine tertiaire de formule NR₁R₂R₃, où R₁, R₂, R₃ sont indépendamment un groupe alkyle en C₁ à C₁₂ linéaire ou ramifié, un groupe alkyl(en C₁ à C₃)aryle(en C₆ à C₁₀), un groupe alcényle en C₁ à C₄, en une proportion en excès de 3 à 15 % par rapport à la quantité molaire de chlorure d'acyle, en maintenant la température de réaction entre 0 et 30 °C ;
c) à ajouter à la solution résultant de l'étape b) un amino-alcool pour donner le N,N'-bis[2,4,6-triiodo-1,3-benzènedicarboxamide] correspondant qui est directement hydrolysé pour déprotéger les groupes hydroxy protégés.

2. Procédé selon la revendication 1, dans lequel ledit milieu de contraste est choisi dans le groupe constitué par : l'Iopromide, l'Iosimide, l'Ioversol, l'Iotriside, l'Iohexol, l'Iopentol, l'Ioxilan, l'Iobitridol, l'Iomeprol.

3. Procédé selon la revendication 1, pour la préparation de (S)-N,N'-bis[2-hydroxy-1-(hydroxyméthyl)-éthyl]-5-[(2-hydroxy-1-oxopropyl) amino]-2,4,6-triiodo-1,3-benzènedicarboxamide, sans isoler aucun produit intermédiaire, comprenant les étapes suivantes du schéma 1 consistant : 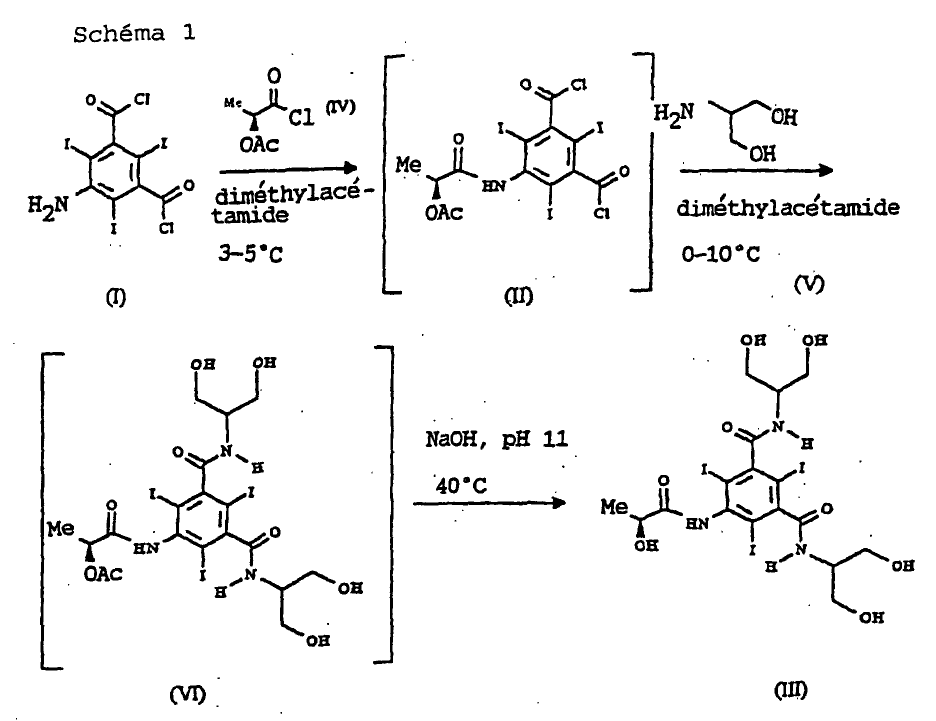
a) à faire réagir le composé (I), le dichlorure d'acide 5-amino-2,4,6-triiodo-1,3-benzènedicarboxylique, avec du chlorure d'acide S-(-)-[2-(acétyloxy)]propionique dans du diméthylacétamide, à une température de 3 à 5°C, pour donner une solution du composé de formule (II), le dichlorure d'acide (S)-5-[[2-acétyloxy)-1-oxopropyl]-amino]-2,4,6-triiodo-1,3-benzènedicarboxylique, dans du diméthylacétamide ;
b) à ajouter une amine tertiaire de formule générale NR₁R₂R₃ à la solution brute résultant de l'étape a), dans laquelle R₁, R₂, R₃ sont indépendamment un groupe alkyle en C₁ à C₁₂ linéaire ou ramifié, un groupe alkyl(en C₁ à C₃)aryle(en C₆ à C₁₀), un groupe alcényle en C₁ à C₄, en une proportion d'excès de 3 à 15 % par rapport à la quantité molaire du chlorure d'acide (S)-(-)-[2-(acétyloxy)]-propionique (IV) de la réaction et dans un laps de temps tel que la température du mélange réactionnel soit maintenue entre 0 et 30 °C ;
c) à ajouter du 2-amino-1,3-propanediol solide à la solution résultant de l'étape b), à une température de 0 à 10 °C, pendant 5 à 20 h, pour donner une solution du composé (VI) qui, sans être isolé, est transformé en composé (III) après élimination du solvant, dilution avec de l'eau et purification chromatographique facultative sur des échangeurs de cations et d'anions et réaction d'hydrolyse dans des conditions basiques par l'addition de NaOH à 30 %, à pH 11, pendant 5 à 10 h ;
d) à isoler le composé de formule (III).

4. Procédé selon la revendication 1 ou 3, dans lequel les amines tertiaires de formule générale NR₁R₂R₃ sont choisies dans le groupe constitué par : la triéthylamine, la tripropylamine, la tributylamine, la diéthylbenzylamine, la diisopropyléthylamine.

5. Procédé selon la revendication 4, dans lequel l'amine tertiaire de formule générale NR₁R₂R₃ est la triéthylamine.

6. Procédé selon la revendication 5, dans lequel la triéthylamine, au terme de la réaction d'amidation, est séparée par filtration sous la forme de chlorhydrate de triéthylamine.

7. Procédé selon la revendication 1 ou 3, dans lequel l'addition de l'amine est effectuée en diluant la solution à la concentration souhaitée pour l'étape suivante à une température comprise entre 0 °C et 30 °C, pendant 2 à 8 h.

8. Procédé selon la revendication 1 ou 3, dans lequel l'amine tertiaire ajoutée est récupérée au terme de l'étape de formation du composé (VI) en partant du composé (II), par élution de la solution brute sur une série d'échangeurs d'ions constitués par une colonne contenant les échangeurs de cations Amberjet® 1200 ou leur équivalent et une colonne contenant les échangeurs d'anions Relite® MG 1 ou leur équivalent.

9. Procédé selon la revendication 8, dans lequel la série d'échangeurs d'ions est remplacée par un lit mixte d'échangeurs d'ions ayant les mêmes caractéristiques.

10. Procédé selon la revendication 1 ou 3, dans lequel l'amine tertiaire ajoutée est récupérée pendant l'étape d'hydrolyse du produit (VI) pour donner le composé (III), en effectuant une séparation des couches dans lesquelles la couche organique correspond à celle contenant l'amine tertiaire.

11. Procédé selon la revendication 10, dans lequel les amines tertiaires ajoutées sont récupérées par distillation azéotropique d'eau, lorsque les aminés le permettent.
